## Europäisches Patentamt
## European Patent Office
## Office européen des brevets

(11) Veröffentlichungsnummer: **0 315 583**
**A2**

(12) # EUROPÄISCHE PATENTANMELDUNG

(21) Anmeldenummer: **88730243.8**

(22) Anmeldetag: **04.11.88**

(51) Int. Cl.⁴: **C 02 F 1/32**
**A 61 L 2/02**

(30) Priorität: **05.11.87 DE 3737850**

(43) Veröffentlichungstag der Anmeldung:
**10.05.89 Patentblatt 89/19**

(84) Benannte Vertragsstaaten:
**AT BE CH DE ES FR GB GR IT LI LU NL SE**

(71) Anmelder: **WEDECO GESELLSCHAFT FÜR
ENTKEIMUNGSANLAGEN MBH
Daimlerstrasse 5
D-4900 Herford (DE)**

(72) Erfinder: **Wedekamp, Horst
Elverdisserstrasse 92
D-4900 Herford (DE)**

(74) Vertreter: **Thömen, Uwe, Dipl.-Ing.
Patentanwalt U. Thömen Zeppelinstrasse 5
D-3000 Hannover 1 (DE)**

(54) **Verfahren zur Entkeimung von Flüssigkeiten und/oder Gasen, sowie Vorrichtung dafür.**

(57) UV-Entkeimungsgeräte werden zur Entkeimung von Flüssigkeiten und/oder Gasen mittels UV-Strahlung eingesetzt. Der wirksame Einsatz zur Entkeimung von Medien mit schwankender bzw. höherer Temperatur, insbesondere von Warmwasser, scheitert daran, daß das Emissionsspektrum der Quecksilber-Niederdruckdampfstrahler direkt von der Betriebstemperatur und auch von der Umgebungstemperatur abhängig ist. Schwankungen der Betriebstemperaur führen einerseits zu einer Verringerung der entkeimenden Strahlung und anderseits zur Auslösung von intralzellulären Reparaturmechanismen.

Bei der Erfindung ist vorgesehen, den Dampfdruck des Quecksilbers der verwendeten Strahler bei Änderungen der Wandtemperatur der Strahler weitgehen konstant zu halten. Dadurch ist das UV-Emissionsspektrum der Quecksilber-Niederdruckdampfstrahler auch bei schwankenden Betriebstemperaturen weitgehend stabilisiert, so daß erstmals die bakteriziede UV-Leistung (Emission der Linie 254 nm) über einen weiten Variationsbereich der Betriebstemperatur nahezu konstant gehalten werden kann. Gleichzeitig wird die bei herkömmlichen Niederdruckstrahlern mit steigender Temperatur einhergehende Erhöhung der UV-A-Emission unterbunden, welche die zellulären Reparaturmechanismen aktiviert.

FIG. 1

# Beschreibung

## Verfahren zur Entkeimung von Flüssigkeiten und/oder Gasen, sowie Vorrichtung dafür

Die Erfindung betrifft ein Verfahren zur Entkeimung gemäß dem Oberbegriff des Patentanspruchs 1, und außerdem befaßt sich die Erfindung mit einer Vorrichtung zur Durchführung des Verfahrens nach dem Oberbegriff des Patentanspruchs 2.

Bekanntlich werden Quecksilber-Niederdruckdampflampen (Hg-Gasentladungslampen) zur Durchführung von photochemischen Reaktionen mit Hilfe von UV-Strahlung verwendet. Dabei ist die photochemische Inaktivierung von Mikroorganismen (Entkeimung) ein besonders wichtiges Anwendungsgebiet. Diese Inaktivierung erfolgt fast ausschließlich durch UV-Strahlung im Bereich um 260 nm. Da das Emissionsspektrum eines Hg-Niederdruckstrahlers bei der Linie 254 nm ihr Maximum besitzt, werden bevorzugt Hg-Niederdruckstrahler zur Entkeimung eingesetzt. Zur Erzielung hoher Entkeimungseffekte ist eine möglichst hohe Strahlungsdichte (UV-Strahlungsleistung pro durchstrahlter Volumeneinheit) anzustreben.

Speziell zur Erzielung hoher Strahlungsdichten wurde das Konzept der positiven Bestrahlungsgeometrie (DE-PS 21 19 961) und der Hg-Niederdruckhochleitungsstrahler (Flachstrahler; DE-PS 28 25 018) entwickelt.

Der besondere Vorteil der positiven Bestrahlungsgeometrie und des Flachstrahlers liegt darin, daß - im Vergleich zu den bisher üblichen UV-Entkeimungsgeräten bzw. zylindrischen Strahlern - Strahlung wesentlich höherer Dichte, d.h., ein wesentlich höherer Entkeimungseffekt erzielt wird.

Alle bisher bekannten UV-Entkeimungsverfahren unter Verwendung von Hg-Niederdruckstrahlern sind nur zur Entkeimung von Fluiden in einem eng begrenzten Temperaturbereich geeignet. Der Grund hierfür liegt darin, daß Temperaturschwankungen im Entkeimungsgerät gemäß der Dampfdruckkurve des Quecksilbers zu Schwankungen des Hg-Partialdrucks im Strahler führen. Dies beeinflußt unmittelbar die Ausbeute an Lichtquanten der Wellenlänge 254 nm durch Anregung in der Gasentladungssäule des Niederdruckstrahlers. Die Folge ist ein Rückgang des Strahlungsflusses bei Abkühlung sowie eine Verschiebung der Lage des Maximums im Emissionsspektrum in den längerwelligen Bereich, z.B. zur Linie 313 nm bei Erwärmung des Strahlers.

Während bei herkömmlichen UV-Entkeimungsgeräten mit Hg-Niederdruckstrahlern dieser Effekt im Kaltwasserbetrieb z.B. durch Erhöhung der Strahlerleistung kompensiert werden kann, ist eine ähnliche Kompensation im Warmwasserbetrieb - also bei höherer Temperatur -bisher nicht möglich.

Der Erfindung liegt die Aufgabe zugrunde, ein UV-Entkeimungsverfahren zu schaffen, das über einen weiten Bereich von Betriebstemperaturen bzw. Umgebungstemperaturen eine sichere Entkeimung gewährleistet. Außerdem soll durch die Erfindung eine Vorrichtung zur Durchführung des Verfahrens geschaffen werden.

Die Lösung dieser Aufgabe erfolgt bei dem im Oberbegriff des Patentanspruchs 1 angegebenen Verfahren durch die Merkmale des kennzeichnenden Teils, und hinsichtlich der Vorrichtung erreicht die Erfindung das Ziel bei der im Oberbgriff des Patentanspruchs 2 genannten Vorrichtung durch die Merkmale des kennzeichnenden Teils des Anspruchs 2.

Bei der Erfindung wird der Anwendungsbereich der UV-Entkeimungsgeräte wesentlich erweitert. Erstmals ist es möglich, auch Wasser mit schwankenden Temperaturen, insbesondere warmes Wasser,wirksam zu entkeimen. Vorteilhaft ist die Verwendung von Zusätzen wie z.B. Indium, die innerhalb des UV-Lampenrohres angeordnet werden und mit dem Quecksilber eine Amalganverbindung eingehen.

Die Dotierung von Hg-Gasentladungsröhren mit Zusätzen ist an sich aus der Beleuchtungstechnik bekannt. Mit der bekannten Art und Anordnung des Zusatzes lassen sich aber bei Hg-Niederdruckdampflampen keine befriedigenden Entkeimungseffekte erzielen. Zahlreiche Versuche haben erwiesen, daß die Fixierung des Zusatzes bzw. der Zusätze an einem oder mehreren definierten Punkten auf der Innenwandung des UV-Lampenrohres, z.B. mittig zwischen den Elektroden, zu einer wesentlich verbesserten Temperaturstabilisierung des Emissionsspektrums im Vergleich zu den herkömmlichen Verfahren der Beleuchtungstechnik führt.

Bei den mit der Erfindung erzielbaren entscheidenden Vorteilen ist auch noch folgender Sachverhalt zu berücksichtigen. Da der Wirkungsquerschnitt der UV-induzierten Keimreduzierung ein ausgeprägtes Maximum im Bereich um 260 nm aufweist, ist eine Stabilisierung des Emissionsmaximums bei 254 nm anzustreben. Hinzu kommt, daß durch UV-Absorption geschädigte Mikroorganismen durch Bestrahlung mit längerwelliger UV-Strahlung intrazelluläre Reparaturmechanismen in Gang setzen, die unter Umständen zu einer Reaktivierung des Organismus führen. Beim Einsatz herkömmlicher Hg-Niederdruckstrahler zur Entkeimung von Wasser der Temperatur von größer als etwa 30° C finden diese Reparaturmechanismen statt, vor allem Dingen bei einer Kreislaufführung des Wassers.

Die dadurch bedingte Anwendungs-Begrenzung auf bestimmte Betriebstemperaturen ist besonders schwer wiegend z.B. bei zentralen Warmwasserversorgungssystemen oder in Betriebswassersystemen, die prozeßbedingt mit schwankenden bzw. erhöhten Temperaturen betrieben werden. Bei der Erfindung treten diese Nachteile jedoch nicht auf, weil der Dampfdruck des Quecksilbers bei Änderungen der Wandtemperatur der Quecksilber-Niederdruckdampflampen weitgehend konstant gehalten wird und somit in gewünschter Weise eine Stabilisierung des Emissionsmaximums bei 254 nm erfolgt.

Durch die Erfindung wird die Zunahme des Anteils von UV-A bei steigender Betriebstemperatur und die dadurch induzierten zellulären Reparaturmechanismen unterbunden. Umgekehrt tritt bei abnehmenden Betriebstemperaturen keine Verminderung der Strahlungsleistung ein. Damit sind die Voraussetzungen geschaffen worden, auch bei stark schwankenden Betriebstemperaturen die zur sicheren Entkeimung erforderlichen Mindest-Bestrahlungsstärken ohne aufwendige Kühl- oder Regelungsvorrichtungen zu gewährleisten. Insbesondere die UV-Entkeimung von Warmwasser wird durch die Erfindung erstmals ermöglicht.

Auch bei der Entkeimung von kaltem Wasser ist die Erfindung von Vorteil, da es nunmehr möglich ist, die Leistung bei niedriger Temperatur - Kaltwasserbetrieb -wegen der Temperaturstabilisierung und der Möglichkeit des Betriebes bei höheren Temperaturen wesentlich zu steigern, womit auch die Betriebstemperatur ansteigt.

In zweckmäßiger Ausgestaltung der Erfindung werden die Zusätze in Form von Indium ortsfest auf der Innenwandung des jeweiligen UV-Lampenrohres befestigt, wobei es vorteilhaft ist, die Zusätze auf einem Trägermaterial in Form eines Goldplättchens anzuordnen, welches seinerseits auf der Innenwandung des UV-Lampenrohres haftet. In Versuchen hat sich gezeigt, daß durch eine solche Anordnung eine besonders sichere Fixierung der Zusätze erreicht wird, und daß vor allem die Zusätze auf dem Goldplättchen verbleiben.

Andere zweckmäßige Ausgestaltungen und vorteilhafte Weiterbildungen der Erfindung sind in den Unteransprüchen und der nachfolgenden Beschreibung angegeben.

Zum besseren Verständnis wird die Erfindung im folgenden anhand der in der Zeichnung dargestellten Ausführungsbeispiele näher erläutert. Es zeigen:

Fig 1 ein Diagramm zweier Dampfdruckkurven von Quecksilber in einer Quecksilber-Niederdruckdampflampe,

Fig 2 die Temperaturabhängigkeit der Emission herkömmlicher Hg-Niederdruckstrahler,

Fig 3 ein elektrisches Prinzipschaltbild eines als Flachstrahler ausgebildeten UV-Lampenrohres,

Fig 4 ein Diagramm zur Verdeutlichung des unterdrückten Anstiegs der langwelligen UV-Strahlung bei Verwendung spezieller Drosseln,

Fig 5 die Temperaturabhängigkeit des Emissionsspektrums bei Dotierung mit einem Indium-Zusatz,

Fig. 6 die Temperaturabhängigkeit des Emissionsspektrums bei Dotierung mit einem Indium-Amalgan, das auf einem Goldplättchen festgeschmolzen ist,

Fig. 7 eine schematische Querschnittsansicht eines Flachstrahlers mit einem Indium-Zusatz,

Fig. 8 eine schematische Querschnittsansicht eines Flachstrahlers mit einer Kühlvorrichtung, und

Fig. 9 eine Querschnittsansicht längs der Schnittlinie II - II gemäß Fig. 8.

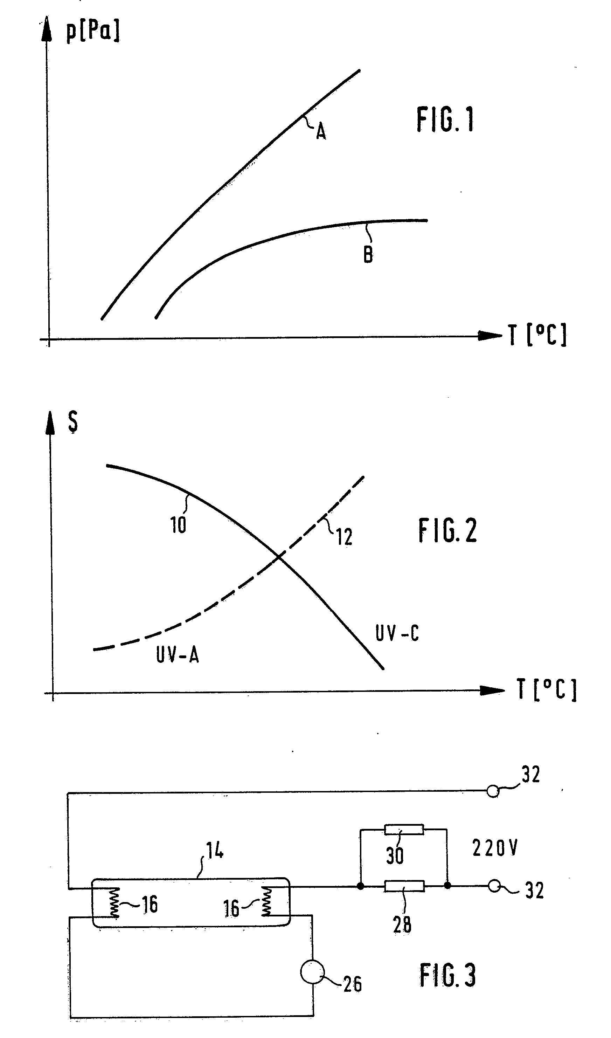

In Fig 1 zeigt die Kurve A den typischen Verlauf des Dampfdrucks des Quecksilbers in einer Hg-Niederdruckdampflampe Auffällig ist dabei die starke Abhängigkeit des Dampfdruckes p von der Temperatur T, die dazu führt, daß sich herkömmliche Hg-Niederdruckdampflampen bei ansteigenden Temperaturen - beispielsweise zur Entkeimung von warmen Wasser mit einer Tempeperatur von größer als 30° C - nicht verwenden lassen.

Durch die Kurve B ist in Fig. 1 dargestellt, daß bei der Erfindung der Dampfdruck des Quecksilbers bei Temperaturänderungen im Vergleich zur herkömmlichen Kurve A weitgehend konstant gehalten wird. Dadurch wird eine Verschiebung des Emissionsspektrums ebenfalls weitgehend vermieden, so daß es nunmehr möglich ist, die Hg-Niederdruckdampflampen bei höheren Temperaturen zu betreiben bzw. die Strahlerleistung bei kaltem Wasser wesentlich zu erhöhen.

Fig. 2 zeigt die Abhängigkeit der Strahlungsintensitäten S der kurzwelligen und langwelligen UV-Strahlung von der Betriebstemperatur eines herkömmlichen Hg-Niederdruckstrahlers. Die Kurve 10 mit der durchgezogenen Linie gibt den Verlauf der für die UV-Entkeimung maßgeblichen UV-Strahlung der Wellenlänge 254 nm wieder (UV-C).

Die gestrichelt darge stellte Linie 12 zeigt, wie das Verhältnis langwelliger Strahlung (UV-A) zur kurzwelligen Strahlung (UV-C) mit steigender Wassertemperatur zunimmt, wenn der Hg-Niederdruckstrahler als Tauchstrahler in Wasser eingetaucht ist. Dieses Verhältnis UV-A zu UV-C ist ein Maß für die relative Stärke von UV-C induzierter Inaktivierung zu UV-A induzierten Zellreparaturen. Es wird deutlich, daß die UV-Entkeimung mit herkömmlichen Hg-Niederdruckstahlern bei Betriebstemperaturen von etwa über 30°C nicht mehr möglich ist.

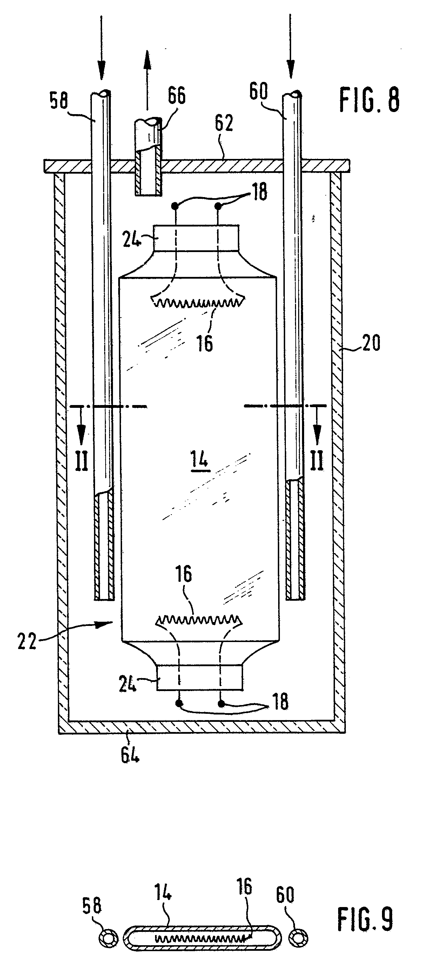

Fig. 7 zeigt einen Flachstrahler 14 mit einer etwa mittig auf der länglichen flachen Seite der Innenwandung angeordneten Indium-Dotierung 44, die mit dem Quecksilber des Flachstrahlers ein Amalgam bildet. Das Indium 44 ist hier mittels eines Goldplättchens 42 innen auf der Quarzwandung des Flachstrahlers 14 befestigt.

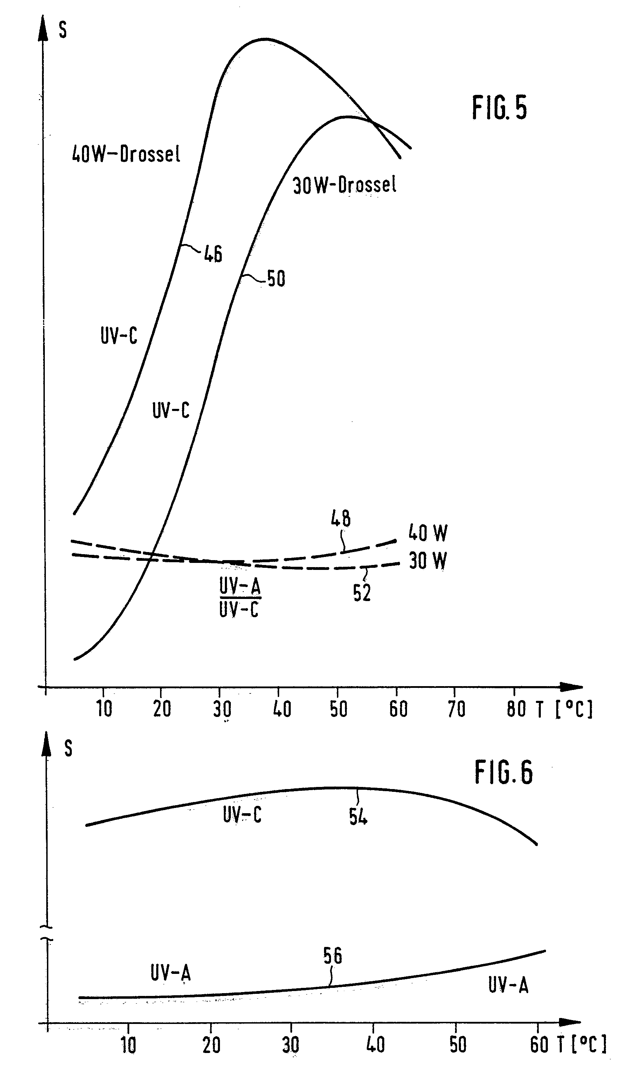

Durch den Indium-Zusatz wird eine Veränderung des Temperaturverhaltens des Flachstrahlers 14 erzielt. Fig. 5 zeigt hierzu die Abhängigkeit der Strahlungsintensität von der Wassertemperatur bei Verwendung verschiedener Drosseln. Die Kurven 46 und 48 beziehen sich auf eine 40 W-Drossel, und die weiteren Kurven 50 und 52 gelten bei Verwendung einer 30 W-Drossel. Die günstigsten Werte wurden mit einem Goldplättchen 42 mit dem Indium 44 etwa mittig zwischen den beiden Elektroden 16 des Flachstrahlers erzielt.

Fig. 6 zeigt die Temperaturabhängigkeit der Stärke der kurzwelligen Strahlung UV-C und der langwelligen Strahlung UV-A bei Verwendung von Amalgam, das auf einem oder mehreren Goldplättchen 42 festgeschmolzen ist. Hier bleibt die Strahlungsintensität des kurzwelligen Anteils UV-C gemäß der Kurve 54 über einen weiten Variationsbereich der Temperatur T nahezu konstant. Es erfolgt also keine temperaturbedingte Verminderung der bakterizieden Wirkung. Auch die reaktivierende Wirkung bei steigenden Fluid/Betriebs-Temperaturen ist unterbunden. Die ausgeprägte Temperaturabhängigkeit mit einem Maximum gemäß Fig. 5 (vgl. dort die Kurven 46 und 50) ist nicht gegeben.

Mittels des Amalgam-dotierten Strahlers gemäß Fig. 6 ist erstmals die Emtkeimung von Fluiden mit schwankenden Betriebstemperaturen möglich.

Fig. 3 zeigt ein elektrisches Anschlußbild eines UV-Lampenrohres 15, welches als Flachstrahler mit zwei endseitigen Elektroden 16 ausgebildet ist. Die Betriebsspannung von 220 V wird über die Anschlüsse 32 zugeführt. Von dort führt eine Verbindung zu der einen Elektrode 16, die über einen bekannten Starter 26 mit der anderen Elektrode 16 verbunden ist. Diese ist ihrerseits über zwei parallel geschaltete Drosseln 28 und 30 mit dem anderen Anschluß 32 verbunden.

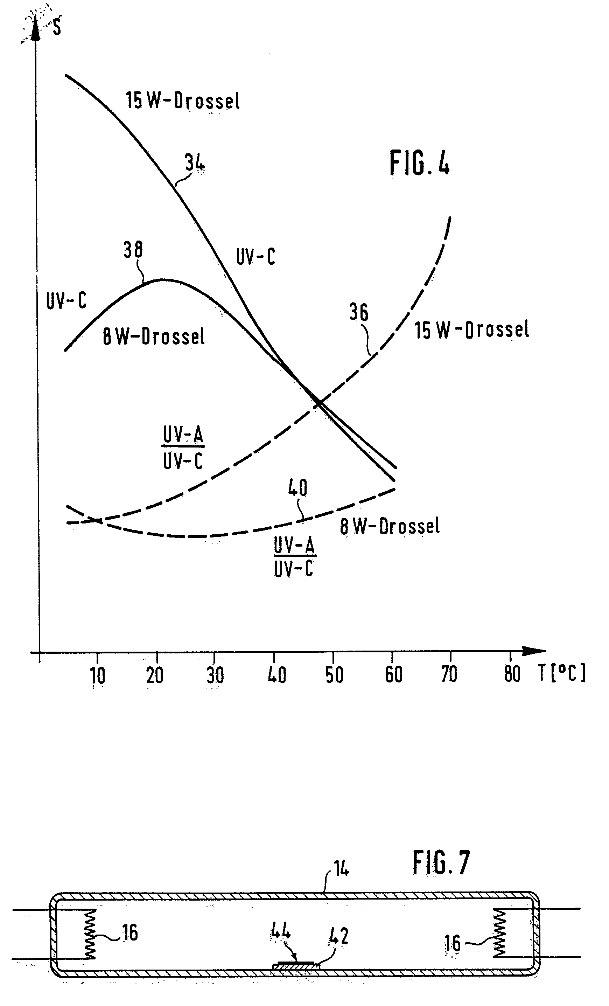

Für die soweit beschriebenen Schaltung sind die Verläufe der Strahlungsintensitäten S in Abhängigkeit von der Betriebstemperatur T in dem Diagramm gemäß Fig. 4 gezeigt, und zwar für Drosseln unterschiedlicher Leistungen. Dabei ist die insgesamt wirksame Leistung der Drosseln 28 und 30 zwischen dem Anschluß 32 und der Elektrode 16 gemeint. An Stelle der beiden parallelen Drosseln 28 und 30 kann man sich auch nur eine einzige Drossel als Ersatz für diese beiden Drosseln denken. Die gezeigte Parallelschaltung in Fig. 3 soll lediglich die Möglichkeit andeuten, bei einer schon vorhandenen Drossel durch Parallelschaltung einen anderen insgesamt wirksamen Leistungswert zu erzielen.

Die Kurve 34 in Fig. 4 gilt für den UV-C Anteil bei einer 15 W-Drossel, und die gestrichelt gezeichnete Kurve 36 gibt bei Verwendung einer gleichen Drossel den Verlauf des Quotienten UV-A/UV-C (und damit auch den Verlauf der langwelligen UV-A Strahlung) wieder.

Wie zu erkennen ist, sinkt der Anleil der kurzwelligen Strahlung UV-C bei zunehmender Betriebstemperatur stark ab, während gleichzeitig der Anteil der langwelligen Strahlung UV-A ansteigt. Dies entspricht den bisher bekannten Verhältnissen, d.h., die Verwendung einer 15 W-Drossel läßt eine wirksame Entkeimung bei höheren Temperaturen nicht zu.

In vorteilhafter Weise wird die Leistung der verwendeten Drossel bzw. Drosseln nun als ein Mittel ausgenutzt, um das unerwünschte Ansteigen des langwelligen Anteiles der Strahlung bei zunehmenden Betriebstemperaturen T weitgehend zu vermeiden. Dies ist in Fig. 4 anhand der Kurven 38 und 40 zu erkennen, die für den Fall maßgeblich sind, daß eine kleinere 8 W-Drossel verwendet wird.

Zwar erfolgt auch hier bei den höheren Betriebstemperaturen T ein Absinken der kurzwelligen Strahlung UV-C, gleichzeitig verdeutlicht jedoch die Kurve 40, daß ein gleichzeitiges Ansteigen der langwelligen Strahlung UV-A nicht mehr gegeben ist. Dadurch wird die schon erwähnte schädliche Reaktivierung der Keime vermieden, so daß es möglicht ist, eine UV-Entkeimung auch bei höheren Betriebsoder Umgebungstemperaturen vorzunehmen.

Die zur Erzielung dieser gewünschtne Wirkung erforderliche Auswahl der verwendeten Drosseln bzw. deren Leistung hängt vom verwendeten Flachstrahler 14 ab und kann leicht in Meßversuchen bestimmt werden. In jedem Fall ist es möglich, durch geeignete Leistungserhöhung oder- Erniedrigung der Drosseln den schädlichen Anstieg der langwelligen Strahlung UV-A in vertretbaren Grenzen zu halten. Somit zeigt Fig. 3 ebenfalls eine Möglichkeit, ein Ansteigen des Anteiles der langwelligen UV-A Strahlung oberhalb 260 nm bei höheren Betriebstemperaturen zu vermeiden.

Besonders vorteilhaft läßt sich die Erfindung unter Verwendung des in Fig. 8 und 9 näher dargestellten Flachstrahlers 22 mit dem UV-Lampenrohr 14 anwenden, welches innerhalb eines Hüllrohres 20 aus Quarz angeordnet ist. Bevorzugt wird dieser Flachstrahler 22 als Tauchlampe eingesetzt, d.h., der Flachstrahler 22 taucht in zu entkeimendes Wasser ein, was durch das Hüllrohr 20 ermöglicht wird.

Das Hüllrohr 20 besitzt eine dem Flachstrahler 22 angepaßte, also ebenfalls länglich-flache Querschnittsform und ist allseits verschlossen, beispielsweise durch einen Deckel 62 am oberen Ende und durch einen Boden 64 am unteren Ende.

Die endseitigen Elektroden 16 des Flachstrahlers 22 besitzen außerhalb des Sockel 24 Elektrodenanschlüsse 18 zur Verbindung mit der zugehörigen elektrischen Schaltung.

Durch den Deckel 62 sind gemäß Fig. 8 zwei Kühlrohre 58 und 60 hindurchgeführt, die sich längs der beiden Schmalseiten des Flachstrahlers 22 erstrecken und beispielsweise mit einem Kühlgas beschickt werden. Das Kühlgas durchströmt die Kühlrohre 58 und 60 und verläßt diese an deren unteren Enden, um dann in den Zwischenraum zwischen dem Flachstrahler 22 und dem Hüllrohr 20 nach oben zurückzuströmen, bis es durch einen im Deckel 62 vorgesehenen Auslaß 66 wieder nach Außen gelangt.

Die hier vorgesehene Kühlung mittels der Kühlrohre 58 und 60 bildet in Ergänzung der Erfindung ebenfalls einen Maßnahme, durch die in der angestrebten Weise ein Ansteigen des Anteiles der langwelligen UV-Strahlung oberhalb 260 nm (UV-A) bei steigender Betriebstemperatur im wesentlichen vermieden wird, so daß die weiter oben geschilderte schädliche Reaktivierung unterbunden ist.

**Patentansprüche**

1. Verfahren zur Entkeimung von kaltem und/oder warmen Flüssigkeiten und/oder Gasen, mit Quecksilber-Niederdruckdampflampen (UV-Strahler), die mit einem für UV-Strahlung durchlässigem Hüllrohr umgeben sind, dadurch gekennzeichnet, daß der Dampfdruck (A) des Quecksilbers bei Änderungen der Wandtemperatur der Quecksilber-Niederdruckdampflampen weitgehend konstant gehalten wird.

2. Vorrichtung zur Durchführung des Verfahrens nach Anspruch 1, mit Quecksilber-Niederdruckdampflampen (UV-Strahler), die mit einem für UV-Strahlung durchlässigem Hüllrohr umgeben sind, da durch gekennzeichnet, daß auf die Innenwandung der Quecksilber-Niederdruckdampflampen (14) mindestens ein fester Zusatz (44) ortsfest aufgebracht ist, der ein Ansteigen der Emission längerwelliger UV-Strahlung über 290 nm bei einer Erhöhung der Wandtemperatur sowie eine Verminderung der Emission kurzwelliger UV-Strahlung bei einer Senkung der Wandtemperatur vermeidet.

3. Vorrichtung nach Anspruch 2, dadurch gekennzeichnet, daß der feste Zusatz durch Indium (44) gebildet ist.

4. Vorrichtung nach Anspruch 2 und/oder 3, dadurch gekennzeichnet, daß Zusätze (44) an einer oder mehreren Stellen ortsfest auf der Innenwandung des UV-Lampenrohres (14) befestigt sind.

5. Vorrichtung nach einem der vorhergehenden Ansprüche 2 - 4, dadurch gekennzeichnet, daß die Zusätze (44) auf einem Trägermaterial (42) aufgebracht sind, welches auf der Innenwandung des UV-Lampenrohres (14) haftet.

6. Vorrichtung nach Anspruch 5, dadurch gekennzeichnet, daß das Trägermaterial durch ein Gold plättchen (42) gebildet ist.

7. Vorrichtung nach einem der vorhergehenden Ansprüche 2 - 6, dadurch gekennzeichnet, daß die Zusätze (44) zwischen den Elektroden (16) auf der Innenwand des UV-Lampenrohres (14) mittig angeordnet sind.

8. Vorrichtung nach einem der vorhergehenden Ansprüche 2 - 6, dadurch gekennzeichnet, daß die Zusätze (44) außermittig auf der Innenwand des UV-Lampenrohres (14) angeordnet sind.

9. Vorrichtung nach einem der vorhergehenden Ansprüche 1 - 8, dadurch gekennzeichnet, daß als Quecksilber-Niederdruckdampflampen Strahler mit flachovalem Querschnitt (Flachstrahler) verwendet sind.

EP 0 315 583 A2

p[Pa]

A

B

FIG.1

T [°C]

S

10

12

UV-A

UV-C

FIG.2

T [°C]

32

14

16   16

30

220V

28

26

32

FIG.3

FIG. 4

S

15 W-Drossel

34

UV-C

38

UV-C

8 W-Drossel

36

15 W-Drossel

$\dfrac{UV-A}{UV-C}$

40

8 W-Drossel

$\dfrac{UV-A}{UV-C}$

10  20  30  40  50  60  70  80  T [°C]

FIG. 7

14

16

44  42

16

FIG. 5

FIG. 6

FIG. 8

FIG. 9